# EUROPEAN PATENT APPLICATION

(11) **EP 4 628 580 A1**
(43) Date of publication of application: **08.10.2025**
(21) Application number: 22967206.8
(22) Date of filing: 01.12.2022
(51) Int. Cl.: C12N 9/16, C07K 19/00, C12N 1/15, C12N 1/19, C12N 1/21, C12N 5/10, C12N 15/09, C12N 15/55, C12N 15/63

(54) **NUCLEASE DOMAIN FOR BREAKING DNA INTO TWO STRANDS**

(71) Applicant: Hiroshima University, Higashi-Hiroshima-shi Hiroshima 739-8511 (JP); Sumitomo Chemical Company, Limited, Tokyo 103-6020 (JP)
(72) Inventor: SAKUMA, Tetsushi, Hiroshima-shi, Hiroshima 739-8511 (JP); YAMAMOTO, Takashi, Hiroshima-shi, Hiroshima 739-8511 (JP); NISHIBORI, Nahoko, Hiroshima-shi, Hiroshima 739-8511 (JP); YOSHIMA, Tadahiko, Osaka-shi, Osaka 554-8558 (JP)
(74) Representative: J A Kemp LLP
(86) International application number: PCT/JP2022/044398
(87) International publication number: WO 2024/116373

(57) **Abstract**

It was found that when two ND1s linked via a linker were used as a nuclease domain of a site-specific DNA-cleaving enzyme, double-strand break was efficiently induced in a target site on DNA.

## Description

### [Technical Field]

The present invention relates to a nuclease domain that induces double-strand break in DNA, and more specifically relates to a fusion nuclease domain comprising two nuclease domains 1 (ND1s) bound via a linker, and the use of the same.

### [Background Art]

Double-strand break in DNA using TALEN, which is an artificial restriction enzyme developed as the second-generation genome editing technology in 2010, requires formation of a dimer of a non-specific DNA cleavage domain of FokI which is a type IIS restriction enzyme, and the TALEN technology requires a pair of TALE repeats. Since a pair of TALENs recognizes and cleaves a target sequence of 30-40 bases in total, the TALEN technology has very high specificity of cleavage, and is thus an excellent genome editing technology that strongly inhibits the generation of off-targets. However, because of the difficulty in simultaneously expressing TALENs of two molecules being a macromolecule of 100 kDa or more, in a cell or in introducing TALEN proteins of the same amount in a cell, and efforts required for the preparation, CRISPR-Cas9 published in 2012 rapidly spread instead of TALEN.

On the other hand, studies to improve TALEN molecules have been continued, and various derivative technologies have been produced, including the development of Platinum TALE, which is highly active TALE. Among these, there have been reports in which two FokI nuclease domains were linked (scFokI), and bound to zinc fingers and TALEs to induce double-strand break (NPLs 1 and 2). This technology made it unnecessary to use a pair of TALEs in the cleavage of a target sequence by TALEN. However, scFokI has a low cleavage activity, and it is difficult to conduct efficient genome editing in human cells. Hence, there has been a demand for further improvement.

Note that the present inventors developed novel two nuclease domains (nuclease domain 1; ND1 and nuclease domain 2; ND2) which are different from the conventional FokI nuclease domains, and succeeded in genome editing of a target site by fusing these nuclease domains with ZF or TALE and utilizing these as a pair of ZFNs or TALENs (PTL 1).

### [Citation List]

### [Patent Literature]

[PTL 1] International Publication No. WO2020/045281

### [Non Patent Literatures]

[NPL 1] Minczuk et al (2008) Nucleic Acids Research, 36(12), 3926-3938
[NPL 2] Mino et al (2009) Journal of Biotechnology, 140(3-4), 156-161

### [Summary of Invention]

### [Technical Problem]

The present invention has been made in view of such circumstances, and an object of the present invention is to provide a nuclease domain that is capable of easily and efficiently inducing double-strand break in DNA.

### [Solution to Problem]

As a result of earnestly conducting studies in order to solve the above-described problems, the present inventors found that when two ND1s linked via a linker were used as a nuclease domain of a site-specific DNA-cleaving enzyme (TALEN as an example), double-strand break was efficiently induced in a target site on DNA. On the other hand, in the case where two ND2s that were linked via a linker were used as a nuclease domain, and in the case where two FokI nuclease domains that were linked via a linker were used as a nuclease domain, double-strand break as in the case of ND1s was not observed. From these facts, it was found that the excellent double-strand break activity of linked ND1s on DNA was not a common event in nuclease domains but is an action unique to ND1s.

In addition, as a result of studying a linker sequence between ND1 and ND1, the present inventors found that it was possible to further enhance the double-strand break activity by adjusting the type or the chain length of the linker sequence, and have completed the present invention.

Therefore, the present invention relates to a fusion nuclease domain comprising two ND1s bound via a linker and the use thereof, and more specifically provides the following.
(1) A fusion nuclease domain comprising two nuclease domains 1 bound via a linker.
(2) A site-specific DNA-cleaving enzyme comprising a DNA-binding domain and the fusion nuclease domain according to (1).
(3) A nucleic acid encoding the fusion nuclease domain according to (1) or the site-specific DNA-cleaving enzyme according to (2).
(4) A vector comprising the nucleic acid according to (3) or a translation product thereof.
(5) A method for producing a cell in which a target DNA is modified, comprising introducing a molecule selected from the following (a) to (c) into a cell:
   (a) the site-specific DNA-cleaving enzyme according to (2),
   (b) a nucleic acid encoding the site-specific DNA-cleaving enzyme of (a), and
   (c) a vector containing the nucleic acid according to (b) or a translation product thereof.
(6) A kit for modifying a target DNA, comprising a molecule selected from the following (a) to (c):
   (a) the site-specific DNA-cleaving enzyme according to (2),
   (b) a nucleic acid encoding the site-specific DNA-cleaving enzyme of (a), and
   (c) a vector containing the nucleic acid according to (b) or a translation product thereof.

### [Advantageous Effects of Invention]

The present invention makes it possible to induce double-strand break in DNA by using a nuclease domain of one molecule, and does not require use of nuclease domains of two molecules for inducing double-strand break in DNA like the conventional techniques. Therefore, by utilizing the nuclease domain of the present invention in a site-specific DNA-cleaving enzyme such as TALEN or ZFN, it is possible to easily and efficiently conduct site-specific DNA editing.

### [Brief Description of Drawings]

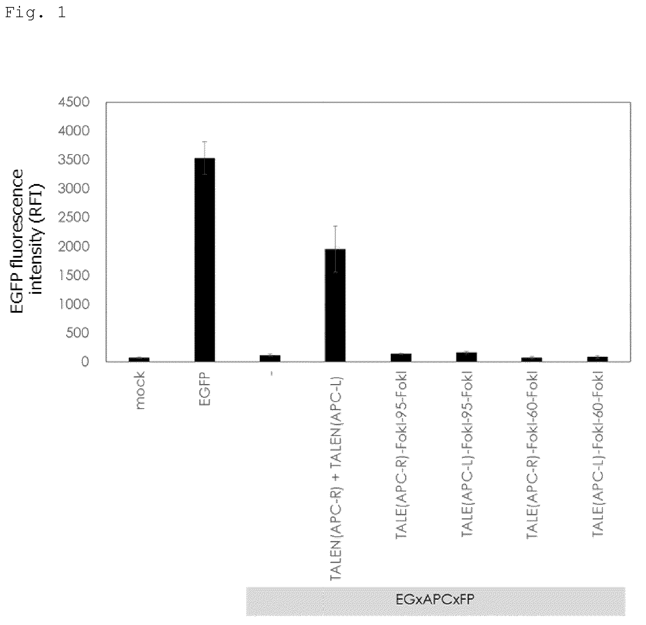

[Fig. 1] Fig. 1 is a graph showing results of detecting DNA cleavage activities of TALENs each containing two FokIs bound via a linker (referred to as "TALE-scFokI") by the SSA assay method. Note that in the figure, "95" indicates an HTS95 linker of 95 amino acid residues, and **"60"** indicates a GGGGS×12 linker of 60 amino acid residues (the same applies hereinafter).

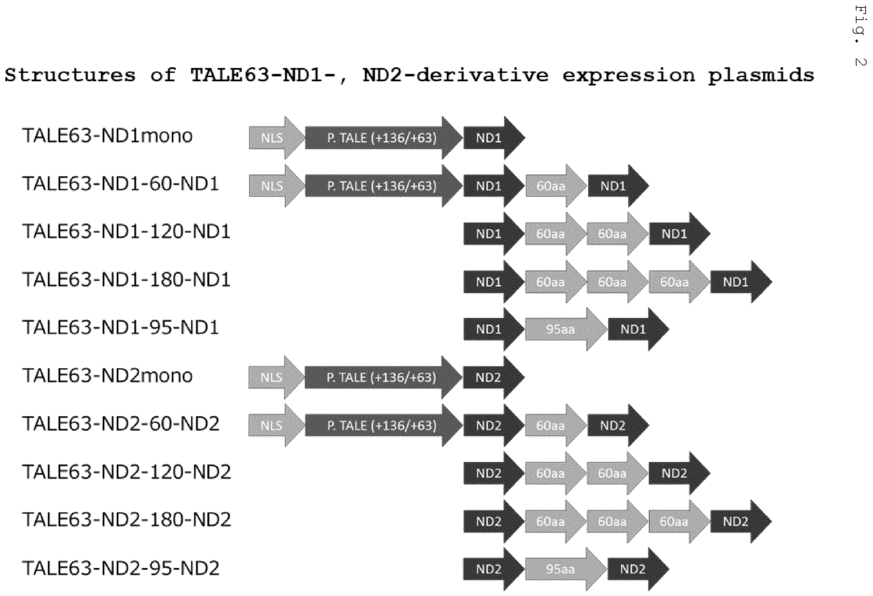

[Fig. 2] Fig. 2 is a diagram showing structures of TALENs each containing two nuclease domains (ND1s or ND2s) bound via a linker (referred to as "TALE-scND1" and " TALE-scND2", respectively). As an example of TALE (DNA-binding domain), the case of using TALE63 is shown. Note that in the figure, "120" indicates a GGGGS×24 linker of 120 amino acid residues, and "180" indicates a GGGGS×36 linker of 180 amino acid residues (the same applies hereinafter).

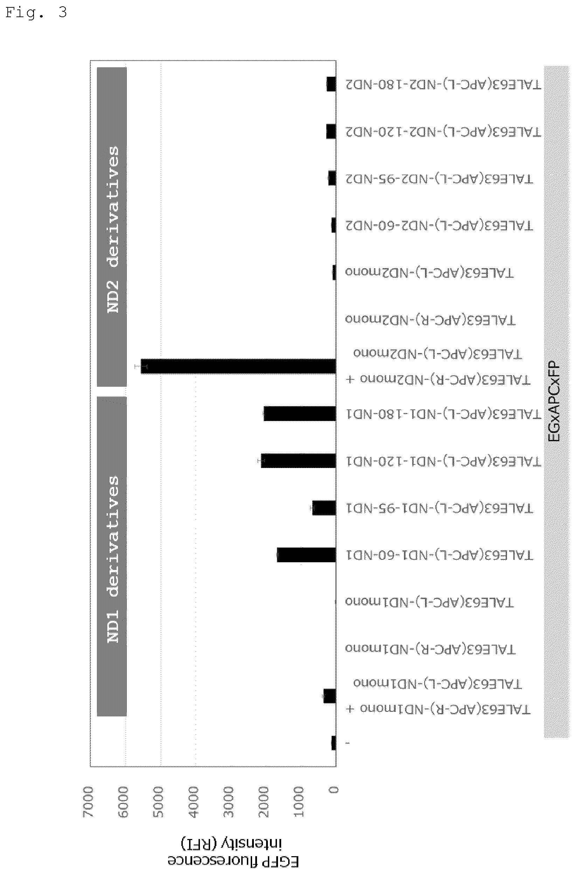

[Fig. 3] Fig. 3 is a graph showing results of detecting DNA cleavage activities by the SSA assay method using the TALE-scND1s and the TALE-scND2s shown in Fig. 2.

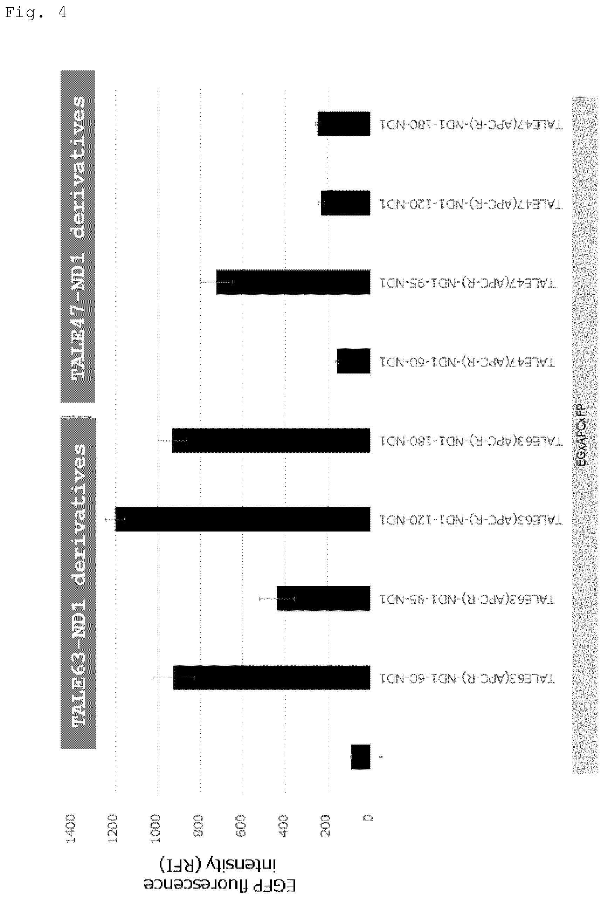

[Fig. 4] Fig. 4 is a graph showing results of evaluating influences of the structures of TALEs and the chain lengths of linkers in the DNA cleavage activity of TALE-scND1 by the SSA assay method. As the TALEs, TALE63 and TALE47 were used.

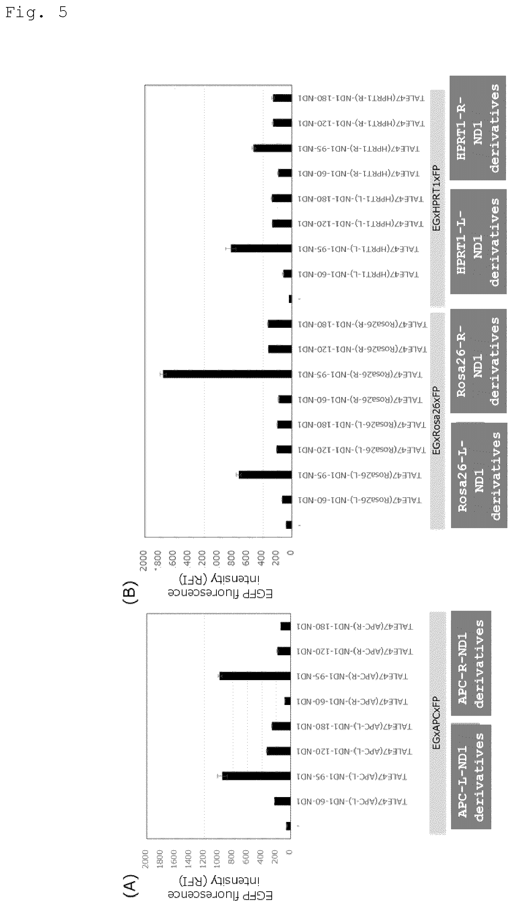

[Fig. 5] Fig. 5 is a graph showing results of evaluating influences of the types of target genes and the chain lengths of linkers in the DNA cleavage activity of TALE-scND1 by the SSA assay method. As the target genes, (A) the APC gene as well as (B) the Rosa26 gene and the HPRT1 gene were used. As the TALE, TALE47 was used.

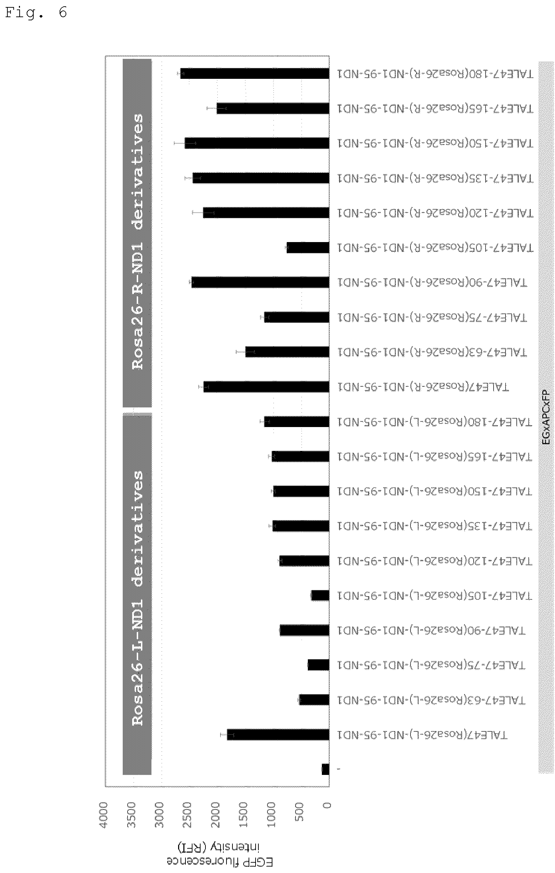

[Fig. 6] Fig. 6 is a graph showing results of evaluating influences of extension of the chain of the C-terminal domain of TALE in the DNA cleavage activity of TALE-scND1 by the SSA assay method. As the target gene, the Rosa26 gene was used. As the TALE, TALE47 was used. As a linker binding two ND1s, an HTS95 linker of 95 amino acid residues was used.

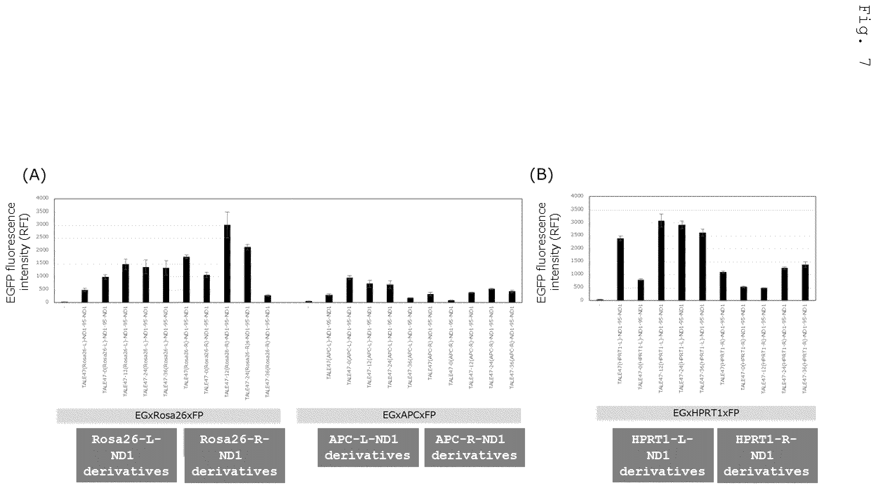

[Fig. 7] Fig. 7 is a graph showing results of evaluating influences of shortening of the chain of the C-terminal domain of TALE in the DNA cleavage activity of TALE-scND1 by the SSA assay method. As the target genes, the Rosa26 gene, the APC gene, and the HPRT1 gene were used. As the TALE, TALE47 was used. As a linker binding two ND1s, an HTS95 linker of 95 amino acid residue was used.

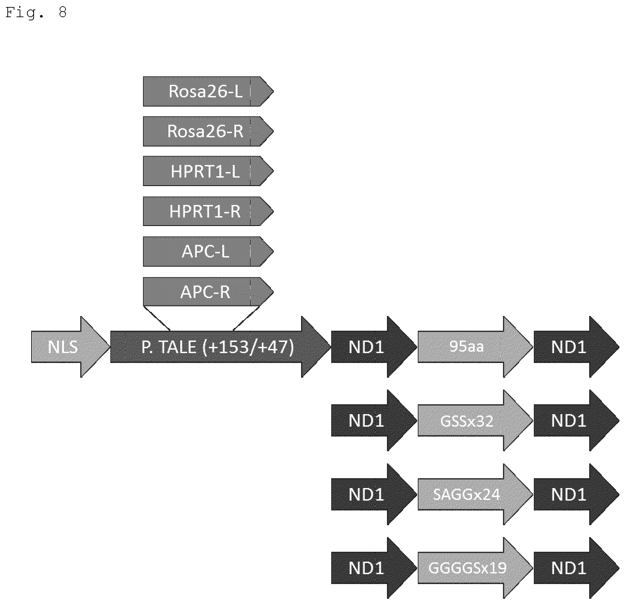

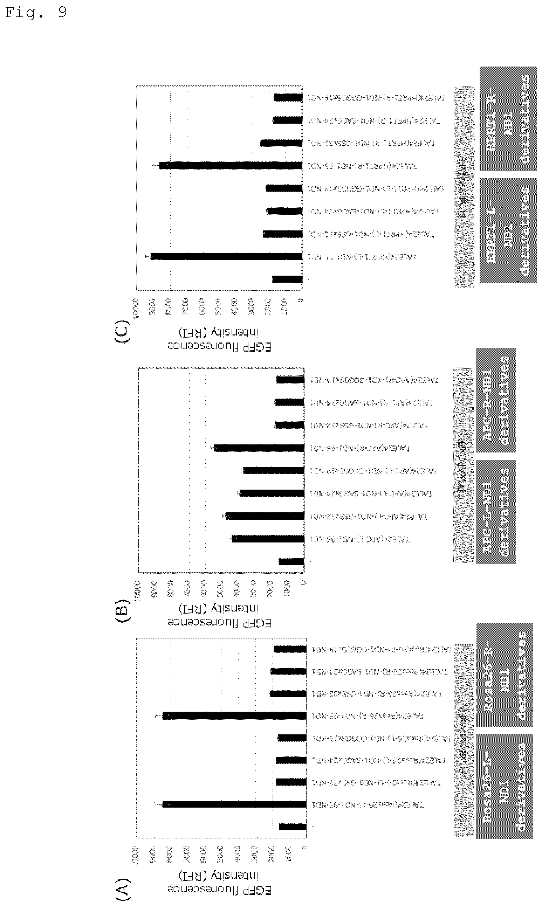

[Fig. 8] Fig. 8 is a diagram showing structures of TALE-scND1s used in experiments of Fig. 9.
[Fig. 9] Fig. 9 is a graph showing results of evaluating influences of the types of linkers in the DNA cleavage activity of TALE-scND1 by the SSA assay method. As the linkers, HTS95 (95 amino acid residues), GSS×32 (96 amino acid residues), SAGG×24 (96 amino acid residues), and GGGGS×19 (95 amino acid residues) were used. As the target genes, (A) the Rosa26 gene, (B) the APC gene, and (C) the HPRT1 gene were used. As the TALE, TALE24 was used.

### [Description of Embodiments]

The present invention provides a fusion nuclease domain (scND1) comprising two ND1s bound via a linker.

The "ND1" in the present invention is one of nuclease domains that the present inventors found by screening homologous sequences having identity with the FokI nuclease domain in the range of 35% to 70% (PTL 1).

The amino acid sequence of the full-length protein containing ND1 (a representative example of which is derived from Bacillus SGD-V-76) is set forth in SEQ ID NO: 97. ND1 is typically a partial peptide corresponding to positions 391 to 585 of SEQ ID NO: 97, and has an identity of 70% with the amino acid sequence of the FokI nuclease domain.

"ND1" in the present invention contains a nuclease domain composed of an amino acid sequence having a high identity with an amino acid sequence corresponding to positions 391 to 585 of SEQ ID NO: 97 as long as when the two molecules of ND1s are bound via a linker, ND1s have a DNA double-strand break activity. Such a nuclease domain includes, for example, ND1 derived from other bacteria and mutants (natural mutants and artificial mutants) of ND1.

Here, the "high identity" means an identity of 85% or more, and is an identity of preferably 90% or more (for example, 95% or more, 96% or more, 97% or more, 98% or more, or 99% or more).

The identity of an amino acid sequence in the present invention is determined by comparing two sequences aligned to maximize sequence matching. Methods for determining the numerical value (%) of the sequence identity are known to those skilled in the art. As an algorithm for obtaining optimum alignment and sequence identity, any algorithm known to those skilled in the art (for example, BLAST algorithm, FASTA algorithm, or the like) may be used. The sequence identity of an amino acid sequence is determined using, for example, sequence analysis software such as BLASTP or FASTA.

The mutants of ND1 include, for example, a nuclease domain composed of an amino acid sequence in which one or more amino acid residues are substituted, deleted, inserted, or added in the amino acid sequence set forth at positions 391 to 585 of SEQ ID NO: 97. Here, "one or more" is, for example, 1 to 30, and preferably 1 to 20 (for example, 1 to 10, 1 to 5, or 1 to 3).

The "linker" for binding two ND1s is not particularly limited in terms of its chain length or type as long as the fusion nuclease domain of the present invention exerts a double-strand break activity. The chain length of the linker is normally 30 to 300 amino acid residues, and preferably 50 to 200 amino acid residues. The type of the linker includes, for example, an HTS95 linker, a GSS linker, an SAGG linker, a GGGGS linker, an HTEN linker, and the like, but the HTS95 linker is preferable. In the case of using these linkers, multiple linkers may be linked and used depending on a required chain length of the linker.

The two ND1s and the linker can be bound at a nucleic acid level and an amino acid level. That is, a nucleic acid encoding the fusion nuclease domain of the present invention can be prepared by linking a nucleic acid encoding ND1, a nucleic acid encoding a linker, and a nucleic acid encoding ND1 through ligation reaction in the order of "the nucleic acid encoding ND1→the nucleic acid encoding a linker→the nucleic acid encoding ND1". By inserting a nucleic acid obtained in this way into an expression vector and expressing the nucleic acid in an appropriate host cell, a fusion nuclease domain in which the two ND1s and the linker are linked at an amino acid level in the order of "ND1→linker→ND1" is obtained. The host cell and the vector are the same as those in the case of the site-specific DNA-cleaving enzyme of the present invention (described later). In addition, it is also possible to prepare the fusion nuclease domain by artificial synthesis based on amino acid sequence information.

It can be evaluated whether or not the fusion nuclease domain has a DNA double-strand break activity from whether or not a site-specific DNA-cleaving enzyme obtained by fusing the fusion nuclease domain with a DNA-binding domain exhibits a site-specific DNA double-strand break activity as described later. For example, as described in Examples of the present application, it is only necessary to evaluate whether or not a site-specific DNA-cleaving enzyme obtained by fusing the fusion nuclease domain with TALE targeting a specific site of a specific gene exhibits a double-strand break activity in the specific site. In this evaluation, the evaluation may be made using an SSA assay system targeting a reporter gene on a plasmid (for example, an EGFP gene in which a TALE recognition sequence is inserted) and using the reporter activity as an index, or the evaluation may be made by preparing a site-specific DNA-cleaving enzyme targeting an endogenous gene in a cell and using modification (mutation) of a base sequence of a target site by the site-specific DNA-cleaving enzyme as an index.

The present invention also provides a site-specific DNA-cleaving enzyme comprising a DNA-binding domain and the above-described fusion nuclease domain. The site-specific DNA-cleaving enzyme of the present invention binds to a target sequence on DNA via the DNA-binding domain and introduces double-strand break in a target cleavage site on the DNA with the fusion nuclease domain.

The "target DNA" of the site-specific DNA-cleaving enzyme of the present invention is preferably double-stranded DNA from the viewpoint that the properties can be most effectively exerted. The double-stranded DNA is not limited, but includes, for example, eukaryotic nuclear genomic DNA, mitochondrial DNA, plastid DNA, prokaryotic genomic DNA, phage DNA, plasmid DNA, and the like.

The DNA-binding domain is not particularly limited as long as the DNA-binding domain is a protein domain capable of specifically binding to a desired DNA sequence (target sequence), but includes, for example, TALE (Transcription Activator-Like Effector), a zinc finger, PPR (Pentatricopeptide repeat), CRISPR/Cas (a complex of Cas protein and guide RNA), and the like.

TALE is a protein that is secreted by proteobacteria in *Xanthomonas* and activates the gene transcription of a host plant. The TALE used in the present invention may contain an N-terminal domain and/or a C-terminal domain in addition to a TALE repeat domain.

The TALE repeat domain is composed of multiple, for example 10 to 30, preferably 13 to 25, and more preferably 15 to 20 tandem repeats (TALE repeats) of TALE sequences which form right-handed superhelix (superhelical). One unit of typical TALE repeats (one TALE sequence) is composed of 33 to 35 amino acids, and recognizes a specific base of DNA by using a variable residue (repeat variable diresidue: RVD) composed of the 12th and the 13th, two amino acid residues. Examples of RVDs that specifically recognize bases include HD which recognizes C, NG which recognizes T, NI which recognizes A, NN which recognizes G or A, NS which recognizes A, C, G, or T, and the like. It is possible to prepare TALE which can recognize and bind to a desired base sequence (TALE recognition sequence) on DNA by artificially linking a TALE sequence which recognizes a specific base based on the DNA recognition mechanism of the TALE repeat domain.

The TALE is preferably a TALE in the form of Platinum TALEN in which amino acids at two specific positions in one unit of TALE repeats change every four units of the TALE repeats (Japanese Unexamined Patent Application Publication No. 2015-33365).

As the N-terminal domain of the TALE, a naturally occurring amino acid sequence (for example, an amino acid sequence of an N-terminal domain contained in pTALETF_v2 (ID: 32185 to 32188) of Addgene) can be used; however, one obtained by appropriately modifying the naturally occurring amino acid sequence may be used as long as it does not exert adverse effects on the function of the site-specific DNA-cleaving enzyme of the present invention (the DNA double-strand break in a target cleavage site). Specific examples of the N-terminal domain of the TALE include, for example, an amino acid sequence of an N-terminal domain contained in ptCMV-136/63-VR-HD (ID: 50699) of Addgene and an amino acid sequence of an N-terminal domain contained in ptCMV-153/47-VR-HD (ID: 50703) of Addgene, but the N-terminal domain is not limited to these.

As the C-terminal domain of the TALE, a naturally occurring amino acid sequence (for example, an amino acid sequence of a C-terminal domain contained in pTALETF_v2 (ID: 32185 to 32188) of Addgene) can be used; however one obtained by appropriately modifying the naturally occurring amino acid sequence may be used as long as it does not exert adverse effects on the function of the site-specific DNA-cleaving enzyme of the present invention (the DNA double-strand break in a target cleavage site). Specific examples of the C-terminal domain include, for example, an amino acid sequence (WT: the number of amino acids=180) of a C-terminal domain contained in pTALETF_v2 (ID: 32185 to 32188) of Addgene, an amino acid sequence (the number of amino acids=63) of a C-terminal domain contained in pTALEN _v2 (ID: 32189 to 32192) of Addgene, and an amino acid sequence (the number of amino acids=47) of a C-terminal domain contained in ptCMV-153/47-VR-NG (ID: 50704) of Addgene, but the C-terminal domain is not limited to these. In fact, in the present Examples, the site-specific DNA double-strand break activity was observed even in the case where a TALE having no C-terminal domain was used, and in the case where a TALE having such a heavy-chain C-terminal domain of 180 amino acid residues was used (Figs. 6, 7).

A DNA-binding domain containing zinc fingers is composed of a zinc finger array (hereinafter, also referred to as "ZFA") consisting of multiple, for example, 3 to 9 zinc fingers. It is known that one zinc finger recognizes a 3-base sequence, and for example, zinc fingers which recognize GNN, ANN, CNN, and the like are known.

The DNA-binding domain containing PPR is composed of multiple, for example, 10 to 30 tandem repeats (PPR repeats). One unit of typical PPR repeats is composed of 35 amino acids, and it is known that one unit of the PPR repeats recognizes one base. In addition, it is also known that while TALE designates a binding base with 2 amino acids present in one unit of the repeat, PPR designates a binding base with 3 amino acids.

CRISPR-Cas is a complex containing a Cas protein and a guide RNA and includes, for example, CRISPR-Cas9, CRISPR-Cpf1 (Cas12a), CRISPR-Cas12b, CRISPR-CasX (Cas12e), CRISPR-Cas14, CRISPR-Cas3, and the like. The CRISPR-Cas used in the present invention is preferably CRISPR-Cas containing Cas (for example, dCas) the nuclease activity of which has been deactivated as a constituent. A Cas mutant the nuclease activity of which has been deactivated is known. In the case of applying the fusion nuclease domain of the present invention to CRISPR-Cas, the fusion nuclease domain normally binds to a protein contained in the CRISPR-Cas. In the case where CRISPR-Cas is a system of type I containing multiple proteins (subunits) as constituents, the fusion nuclease domain of the present invention may be fused to a subunit that does not have a nuclease activity (for example, a subunit contained in Cascade in CRISPR-Cas3). In this case, a subunit having a nuclease activity (for example, Cas3 in CRISPR-Cas3) can be removed from the constituents of the CRISPR-Cas system.

In the site-specific DNA-cleaving enzyme of the present invention, the fusion nuclease domain and the DNA-binding domain may be bound directly, or may be bound via a linker. In the case where a linker is present, the linker is not particularly limited in terms of its chain length or type as long as the fusion nuclease domain of the present invention can exert a double-strand break activity. The chain length of the linker is normally 2 to 180 amino acid residues, and preferably 2 to 120 amino acid residues. The type of the linker includes, for example, a HTS95 linker, a GSS linker, an SAGG linker, a GGGGS linker, and an HTEN linker, but the linker is not limited to these. In the case of using these linkers, multiple linkers may be linked and used depending on a required chain length of the linker.

The fusion nuclease domain may be bound to the **N-**terminal side or the C-terminal side of a DNA-binding domain. In addition, a sandwich type may be configured by arranging DNA-binding domains on both ends of the fusion nuclease domain (see Mori et al (2009) Biochemical and Biophysical Research Communications, 390(3), 694-697 regarding the sandwich type). Moreover, FLAG-tags for purification or detection, or various localization signals (for example, a nuclear localization signal, a mitochondrial localization signal, a plastid localization signal, and the like) may be attached.

A target sequence to be recognized by the DNA-binding domain of the site-specific DNA-cleaving enzyme of the present invention is any sequence on DNA. In the conventional ZFN and TALEN which are used as a pair (2 molecules), it is necessary that target sequences are 2 sequences sandwiching a spacer sequence. However, the site-specific DNA-cleaving enzyme of the present invention can be used as one molecule, which is also advantageous in that the limitation in targeting is low.

The site-specific DNA-cleaving enzyme of the present invention can be produced by a method known to those skilled in the art. For example, such a method includes a method for expressing the site-specific DNA-cleaving enzyme in a host cell by inserting a nucleic acid encoding the site-specific DNA-cleaving enzyme of the present invention into an appropriate expression vector and introducing the expression vector into a host cell, and a method for artificially synthesizing the site-specific DNA-cleaving enzyme based on amino acid sequence information of the site-specific DNA-cleaving enzyme. The expression vector may be selected as appropriate from vectors used in the art, and includes, for example, plasmid vectors, viral vectors, phage vectors, phagemid vectors, BAC vectors, YAC vectors, MAC vectors, HAC vectors, and the like. The host cell into which the expression vector is introduced may be selected as appropriate considering the compatibility with the expression vector, and includes, for example, cells of prokaryotes such as Escherichia coli, actinomycetes, and archaea, and eukaryotes such as yeast, sea urchins, silkworms, zebrafish, mice, rats, frogs, tobacco, Arabidopsis thaliana, and rice.

The present invention provides a nucleic acid encoding the above-described fusion nuclease domain and a nucleic acid encoding the above-described site-specific DNA-cleaving enzyme. Here, the "nucleic acid" encompasses both DNA and RNA (mRNA). In the above-described nucleic acid, codon optimization may be conducted for the purpose of enhancing the expression efficiency in a cell, and the like.

The present invention also provides a method for producing a cell in which a target DNA is modified, comprising introducing the site-specific DNA-cleaving enzyme of the present invention, a nucleic acid encoding the site-specific DNA-cleaving enzyme, or a vector containing the nucleic acid or a translation product thereof into a cell.

The "modification" of a target DNA in the present invention includes deletion, insertion, substitution, or a combination of these of at least one nucleotide in the target DNA.

The introduction of the above-described molecule into a cell may be physical introduction, or introduction via infection of a virus or an organism, or the like, and various methods known in the art can be used. The physical introduction method is not particularly limited, but includes, for example, an electroporation method, a particle gun method, a microinjection method, a lipofection method, a protein transduction method, and the like. The introduction method via infection of a virus or an organism, or the like is not particularly limited, but includes, for example, virus transduction, an agrobacterium method, phage infection, conjugation.

The above-described introduction uses a vector as appropriate. The vector may be selected as appropriate depending on the type of a molecule or a cell for introduction and is not particularly limited, but includes, for example, the above-described plasmid vectors, viral vectors, phage vectors, phagemid vectors, BAC vectors, YAC vectors, MAC vectors, HAC vectors, and the like. In addition, the vector also includes vectors such as liposome and lentivirus capable of carrying mRNAs (transcription products), proteins (translation products), and the like, and peptide vectors such as cell-permeable peptides capable of introducing fused or bound molecules into cells. Hence, the "vector containing a nucleic acid encoding the site-specific DNA-cleaving enzyme or a translation product thereof" in the present invention encompasses not only vectors such as the above-described plasmid vectors in which DNA encoding the site-specific DNA-cleaving enzyme is inserted but also vectors such as the above-described liposomes holding mRNAs (transcription products) and proteins (translation products).

The cells into which the above-described molecules are inserted may be either prokaryotic cells or eukaryotic cells. The cells include, for example, bacteria, archaea, yeast, plant cells, insect cells, and animal cells (for example, human cells, non-human cells, non-mammalian vertebrate cells, invertebrate cells, and the like). The cells may also be in vivo cells or isolated cells, and may be primary cells or cultured cells. The cell may also be somatic cells, germ cells, or stem cells.

Prokaryotes from which the above-described cells are derived include, for example, Escherichia coli, actinomycetes, archaea, and the like. In addition, eukaryotes from which the above-described cells are derived include, for example, fungi such as yeast, mushrooms, and molds, echinoderms such as sea urchins, sea stars, and sea cucumbers, insects such as silkworms and flies, fish such as tuna, bream, pufferfish, mackerel tuna, and zebrafish, rodents such as mice, rats, guinea pigs, hamsters, and squirrels, even-toed ungulates such as cows, wild boars, pigs, sheep, and goats, odd-toed ungulates such as horses, reptiles such as lizards, amphibians such as frogs, Lagomorphs such as rabbits, Carnivora such as dogs, cats, and ferrets, birds such as chickens, ostriches, and quails, and plants such as tobacco, Arabidopsis thaliana, rice, corn, banana, peanut, sunflower, tomato, melon, rapeseed, wheat, barley, potato, soybean, cotton, Japanese morning glory, Persian cyclamen, and carnation. The "animal cells" include, for example, embryonic cells of embryos at various stages (for example, such as 1-cell stage embryo, 2-cell stage embryo, 4-cell stage embryos, 8-cell stage embryos, 16-cell stage embryos, and mulberry stage embryos); stem cells such as induced pluripotent stem (iPS) cells, embryonic stem (ES) cells, and hematopoietic stem cells; somatic cells such as fibroblasts, hematopoietic cells, neurons, muscle cells, osteocytes, hepatocytes, pancreatic cells, brain cells, and kidney cells; fertilized eggs, and the like.

The target DNA may be any gene in the genomic DNA in the above-described cells or DNA in an extra gene region. In order to cleave the target site in the target DNA, the site-specific DNA-cleaving enzyme of the present invention is designed so that the DNA-binding domain contained in the site-specific DNA-cleaving enzyme of the present invention binds to a sequence in the vicinity of the target cleavage site (selected as the target sequence of the DNA-binding domain). As a result, the sequence of the target cleavage site in the target DNA is cleaved, for example, the expression of the gene is reduced or eliminated, or the function of the gene is reduced or no function is expressed.

In the method of the present invention, a donor polynucleotide may be introduced into a cell in addition to the above-described site-specific DNA-cleaving enzyme. The donor polynucleotide contains at least one donor sequence containing modification desired to be introduced at the target cleavage site. The donor polynucleotide can be designed by those skilled in the art as appropriate based on technologies known in the art. When a donor polynucleotide is present in the method of the present invention, homologous recombination repair occurs at the target cleavage site, and the donor polynucleotide is inserted into the site or the site is substituted with the donor sequence. As a result, the desired modification is introduced at the target cleavage site.

In the case where no donor polynucleotide is present in the method of the present invention, the target cleavage site is repaired primarily by non-homologous end joining (NHEJ). Since NHEJ is error prone, deletion, insertion, or substitution of at least one nucleotide, or a combination thereof can occur during repair of the cleavage. In this way, the target DNA is modified at the target cleavage site.

In addition, the present invention provides a kit for modifying a target DNA, comprising the above-described site-specific DNA-cleaving enzyme, a nucleic acid encoding the site-specific DNA-cleaving enzyme, or a vector containing the nucleic acid or a translation product thereof. The kit may include one or a plurality of additional reagents, and the additional reagents include, for example, a diluted buffer solution, a reconstitution solution, a wash buffer solution, a nucleic acid transfection reagent, a protein transfection reagent, and a control reagent, but the reagent is not limited to these. The kit usually comes with an instruction of use.

### Examples

### 1. Method

### (1) Preparation of TALE vector sets and TALE expression plasmids.

For the sequences and configurations of TALE vector sets, materials were constructed from scratch in accordance with a document (Sakuma et al (2013) Scientific Reports, 3, 1-8). First, sequences having recognition sites of restriction enzyme BsAI added to both ends of each base sequence were prepared by artificial gene synthesis for module plasmids (16 types in total) corresponding to various RVDs of HD, NG, NI, and NN while holding a variation called non-repeat-variable di-residues (non-RVDs). These were inserted into pEX-A2J2 (Eurofins Genomics, Tokyo, Japan) to prepare module plasmid sets (pEX1HD-pEX4HD, pEX1NG-pEX4NG, pEX1NI-pEX4NI, and pEX1NN-pEX4NN). Next, DNA sequences FUS2_axx (7 types) and b(1-4) (4 types) forming array plasmids were prepared by artificial gene synthesis. These were inserted into pCR8/GW/TOPO (Thermo Fisher Scientific, Waltham, MA, USA) to prepare pCR8_FUS2_axx and pCR8_FUS b(1-4), which were used as trapping vectors for the initial assembly step in the Platinum Gate system described in the above-described document of Sakuma et al. Final vectors were prepared by inserting 3 types of TALE structures, WT, +136/+63, and +153/+47, prepared by artificial gene synthesis and the final repeat into pcDNA3.1s obtained by removing a drug-resistant gene expression unit from pcDNA3.1(+) (Thermo Fisher Scientific). TALE expression plasmids were prepared by the Golden Gate method in accordance with the above-described document of Sakuma et al. by using the above-described plasmids.

### (2) Preparation of single-chain FokI, single-chain ND1, and single-chain ND2 expression plasmids

An artificial gene (FokI-95-FokI) in which two partial genes encoding nuclease domains of FokI genes were linked via a DNA sequence encoding the HTS95 amino acid sequence (Sun, N., & Zhao, H. (2014) Molecular BioSystems, 10(3), 446-453) was synthesized. In addition, an artificial gene (FokI-GGGGSx12-FokI) in which the HTS95 amino acid sequence was substituted with a DNA sequence encoding a linker of GGGGSx12 (60 amino acid residues in total) was also synthesized. For ND1 genes and ND2 genes, ND1-60-ND1 in which a linker of GGGGSx12 (60 amino acid residues in total) was inserted between partial genes encoding the nuclease domains of ND1s, and ND2-95-ND2 in which a HTS95 amino acid sequence was inserted between partial genes encoding the nuclease domains of ND2s were artificially synthesized. Note that the artificial synthesis was conducted by adding a restriction enzyme AleI/XmaI site before, and a restriction enzyme PshAI/SacII site after, the linker sequences of GGGGSx12 and HTS95 as consensus sequences. These artificially synthesized genes were inserted into pEX-A2J2 (Eurofins Genomics, Tokyo, Japan).

Next, ND1-60-ND1/pEX-A2J2 and ND2-95-ND2/pEX-A2J2 were cleaved with restriction enzymes AleI and PshAI, and a 295bp DNA fragment (95aa) encoding the HTS95 amino acid sequence, a 190bp DNA fragment (60aa) encoding the linker of GGGGSx12, and a fragment of each remaining vector portion were isolated. The 60aa was linked by ligation reaction using T4 DNA ligase, and then cleaved with restriction enzymes XmaI and SacII, and DNA fragments thus obtained were separated through agarose gel electrophoresis to isolate and purify a 380bp DNA fragment (120aa) and a 570bp DNA fragment (180bp). Fragments of 95aa, 120aa, and 180aa were each inserted between fragments of vector portions obtained by cleaving ND1-60-ND1/pEX-A2J2 with restriction enzymes AleI and PshAI, and also fragments of 60aa, 120aa, and 180aa were each inserted between fragments of vector portions obtained by cleaving ND2-95-ND2/pEX-A2J2 with restriction enzymes AleI and PshAI to obtain plasmids ND1-95-ND1/pEX-A2J2, ND1-120-ND1/pEX-A2J2, ND1-180-ND1/pEX-A2J2, ND2-60-ND2/pEX-A2J2, ND2-120-ND2/pEX-A2J2, and ND2-180-ND2/pEX-A2J2.

Next, a TALE63-scFokI expression plasmid, a TALE63-scND1 expression plasmid, and a TALE63-scND2 expression plasmid in which the respective sequences of the above-described scFokI, scND1, and scND2 were linked to downstream of TALE (+136/+63) were prepared by a method described below. The respective sequences of scFokI, scND1, and scND2 were amplified by PCR using FokI-95-FokI/pEX-A2J2, FokI-GGGGSx12-FokI/pEX-A2J2, ND1-95-ND1/pEX-A2J2, ND1-60-ND1/pEX-A2J2, ND1-120-ND1/pEX-A2J2, ND1-180-ND1/pEX-A2J2, ND2-95-ND2/pEX-A2J2, ND2-60-ND2/pEX-A2J2, ND2-120-ND2/pEX-A2J2, and ND2-180-ND2/pEX-A2J2 of the above-mentioned plasmids as templates. These were inserted into downstream of +136/+63, which is the Platinum TALE structure of the TALE expression plasmid by an in-fusion method (TaKaRa Bio Inc, Shiga, Japan) to prepare a TALE63-scFokI expression plasmid, a TALE63-scND1 expression plasmid, and a TALE63-scND2 expression plasmid. Primers used in the preparation of these are shown in Table 1.

**[Table 1]**

| **Products** | **Template** | **Forward Primer** | **SEQ ID NO** | **Reverse Primer** | **SEQ ID NO** |
|---|---|---|---|---|---|
| scFolI | Synthetic scFokI | FokI_FW | 1 | FokI_RV | 2 |
| TALE63 expression vector | TALE136-63VRNN/pcDNA3.1s | TALE63-RinfFokI | 3 | Vector-FinfFokI | 4 |
| scND1 | scND1/pEX-A2J2 | ND1F_inf63-2 | 5 | ND1R_inf63 | 6 |
| scND2 | scND2/pEX-A2J2 | ND2F_inf63 | 7 | ND2R_inf63 | 8 |
| ND1 monomer | scND1/pEX-A2J2 | ND1F_inf63-2 | 5 | NDlmonoR_inf63 | 9 |
| ND2 monomer | scND2/pEX-A2J2 | ND2F_inf63 | 7 | ND2monoR_inf63 | 10 |
| TALE63 expression vector | TALE136-63VRNN/pcDNA3.1s | TALE_63_R | 11 | TALEvector_F | 12 |
| TALE47 expression vector | TALE153-47VRNN/pcDNA3.1s | TALE_47_R | 13 | TALEvector_F | 12 |

Note that the base sequences and amino acid sequences of the N-terminal domain and the C-terminal domain of the TALE+136/+63 are shown in SEQ ID NOs: 64 to 67, the base sequence and the amino acid sequence of each scFokI are shown in SEQ ID NOs: 60 to 63, the base sequence and the amino acid sequence of each scND1 are shown in SEQ ID NOs: 72 to 79, and the base sequence and the amino acid sequence of each scND2 are shown in SEQ ID NOs: 80 to 87. In the amino acid sequence (SEQ ID NO: 67) of the C-terminal domain of the TALE+136/+63, when scFokI is used as a fusion nuclease domain, two amino acids "LK" are added to the C-terminal side.

### (3) Preparation of reporter plasmids for single strand annealing (SSA) assay

For a reporter plasmid for SSA assay, first, amplicons of two fragments on the N-terminal side and the C-terminal side of the EGxxFP sequence were obtained by PCR using, as a template, totally synthesized EGFP cDNA, with reference to sequence information described in a document (Mashiko et al (2013) Scientific Reports, 3, 1-6). Subsequently, amplicons were obtained by conducting PCR again using primers to which linker sequences between the N-terminal side and the C-terminal side were added. Thereafter, these were inserted into a restriction enzyme BamHI/EcoRV site of pcDNA3.1s by the in-fusion method to prepare plasmids (the plasmids thus obtained are reffered to as "pcEGxxFP") . Primers used in the preparation of pcEGxxFP are shown in Table 2.

**[Table 2]**

| **Products** | **Template** | **Forward Primer** | **SEQ ID NO** | **Reverse Primer** | **SEQ ID NO** |
|---|---|---|---|---|---|
| EGFP-N terminal | Synthetic EGFP | EGFP_F+bam | 14 | EGFP_600R | 15 |
| EGFP-N terminal + linker | EGFP-N terminal | EGFP_F+bam | 14 | EGFP _600R+linker | 16 |
| EGFP-C terminal | Synthetic EGFP | EGFP_120F | 17 | EGFP_R+rv | 18 |
| EGFP-C terminal + linker | EGFP-C terminal | linker+EGFP_120F | 19 | EGFP_R+rv | 18 |

Each target sequence to be used in the SSA assay was isolated from the genome by PCR, and these were inserted into the restriction enzyme BamHI/EcoRI site in a linker inserted between the N-terminal side and the C-terminal side of EGFP in pcEGxxFP by the in-fusion method to prepare each reporter plasmid for the SSA assay. The target sequences (APC, Rosa26, and HPRT1) used in the SSA assay evaluation are shown in SEQ ID NOs: 94 to 96.

### (4) Preparation of plasmids for studying TALE C-terminal length

The chain length between scND1 and TALE repeat was studied by changing the C-terminal length starting from the C terminus of TALE+153/+47. To extend the chain length, PCR was conducted using TALE WT as a template to prepare 9 types of TALE C-terminal regions having chain lengths of from 63 residues to 180 residues, which were linked to the N-terminal region of TALE+153/+47. To shorten the chain length, PCR was conducted using TALE+153/+47 as a template to prepare 4 types of TALEs having chain lengths of from 0 residues to 36 residues. Primers used in the preparation of expression plasmids of the respective TALEs having different C-terminal lengths are shown in Table 3. In addition, the base sequences and the amino acid sequences of the N-terminal domain and the C-terminal domain of TALE+153/+47 are shown in SEQ ID NOs: 68 to 71.

**[Table 3]**

| **Products** | **Template** | **Forward Primer** | **SEQ ID NO** | **Reverse Primer** | **SEQ ID NO** |
|---|---|---|---|---|---|
| TALE47 N-terminal + β-lactamase | TALE47_ND1-95-ND1/pcDNA3.1s | TALE-CR | 20 | Amp_R | 21 |
| ND1-95-ND1 + β-lactamase | TALE47_ND1-95-ND1/pcDNA3.1s | ND1-N_F | 22 | Amp_F | 23 |
| TALE-C terminal-63aa | TALE136-WIVRNM/pcDNA3. 1s | WT-CF | 24 | WT-63R_infND1 | 25 |
| TALE-C terminal-75aa | TALE136-WTVRNN/pcDNA3. 1s | WT-CF | 24 | WT-75R_infND1 | 26 |
| TALE-C terminal-90aa | TALE136-WTVRNN/pcDNA3. 1s | WT-CF | 24 | WT-90R_infND1 | 27 |
| TALE-C terminal-105aa | TALE136-WTVRNN/pcDNA3.1s | WT-CF | 24 | WT-105R_infND1 | 28 |
| TALE-C terminal-120aa | TALE136-WTVRNN/pcDNA3.1s | WT-CF | 24 | WT-120_infND1 | 29 |
| TALE-C terminal-135aa | TALE136-WTVRNN/pcDNA3.1s | WT-CF | 24 | WT-135R infND1 | 30 |
| TALE-C terminal-150aa | TALE136-WTVRNN/pcDNA3.1s | WT-CF | 24 | WT-150R_infND1 | 31 |
| TALE-C terminal-165aa | TALE136-WIVRNM/pcDNA3. 1s | WT-CF | 24 | WT-165R_infND1 | 32 |
| TALE-C terminal-180aa | TALE136-WTVRNN/pcDNA3. 1s | WT-CF | 24 | WT-180R_infND1 | 33 |
| TALE47 C-terminal-0aa + β-lactamase | TALE47_ND1-95-ND1/pcDNA3.1s | TALE-0_R_infND1 | 34 | Amp_R | 21 |
| TALE47 C-terminal-12aa + β-lactamase | TALE47_ND1-95-ND1/pcDNA3.1s | TALE-12_R_infND1 | 35 | Amp_R | 21 |
| TALE47 C-terminal-24aa + β-lactamase | TALE47_ND1-95-ND1/pcDNA3.1s | TALE-24_R_infND1 | 36 | Amp_R | 21 |
| TALE47 C-terminal-36aa + β-lactamase | TALE47_ND1-95-ND1/pcDNA3.1s | TALE-36 R infND1 | 37 | Amp_R | 21 |

### (5) Preparation of flexible linkers

Three types of flexible linkers GSS, SAGG, and GGGGS were prepared such that the flexible linkers could be inserted into target sequences in a form that allowed the number of repeats to be adjusted. First, linker sequence GSSx4 adaptor, SAGGx3 adaptor, and GGGGSx3 adaptor (forward and reverse for each) in which a 5' protruding cleavage sequence having restriction enzyme XmaI at the 5' end and BamHI or MroI at the 3' end was disposed were synthesized. Primers used in the preparation of these are shown in Table 4.

**[Table 4]**

| **Products** | **Forward Oligo.** | **SEQ ID NO** | **Reverse Oligo.** | **SEQ ID NO** | **5' end** | **3' end** |
|---|---|---|---|---|---|---|
| GSSx4 adaptor | GSSx4+5'ad._F | 38 | GSSx4+5'ad._R | 39 | XmaI | MroI |
| SAGGx3 adaptor | SAGGx3+5'ad._F | 40 | SAGGx3+5'ad._R | 41 | XmaI | BamHI |
| GGGGSx3 adaptor | GGGGSx3+5'ad. F | 42 | GGGGx3+5'ad._R | 43 | XmaI | MroI |
| GSSx4 linker | GSSx4_F | 44 | GSSx4_R | 45 | XmaI | MroI |
| GSSx7 linker | GSSX7_F | 46 | GSSx7_R | 47 | XmaI | MroI |
| SAGGx3 linker | SAGGx3_F | 48 | SAGGx3_R | 49 | BglII | BamHI |
| SAGGx4 linker | SAGGx4_F | 50 | SAGGx4_R | 51 | BglII | BamHI |
| GGGGSx3 linker | GGGGSx3_F | 52 | GGGGSx3_R | 53 | XmaI | MroI |
| GGGGSx4 linker | GGGGSx4_F | 54 | GGGGSx4_R | 55 | XmaI | MroI |

The 5' end of each oligo was phosphorylated and annealed, and then, it was inserted into plasmids each having XmaI, BamHI, MroI in one location (for example, since there are XmaI, BamHI, MroI each in one location in the 60aa linker sequence of TALE63-ND1-60-ND1, TALE63-ND1-60-ND1/pcDNA3.1s was used as a temporal vector in the present Examples). Thus, GSS adaptor vector, SAGG adaptor vector, and GGGGS adaptor vector were obtained.

In addition, linker sequence oligos GSSx4, GSSx7, SAGGx3, SAGGx4, GGGGSx3, and GGGGSx4 (forward and reverse for each) in each of which a 5' protruding cleavage sequence having restriction enzyme BglII at the 5' end and BamHI at the 3' end or XmaI at the 5' end and MroI at the 3' end was disposed as a flexible linker repeat sequence to be inserted in each adaptor vector were synthesized (Table 4).

The 5' end of each oligo was phosphorylated and annealed, and then, ligation reaction was conducted, followed by double digestion with BglII and BamHI or XmaI and MroI, and fragments thus having a target number of repeats were collected through agarose gel electrophoresis. For example, in the case of GSSx4 repeats, repeat sequences such as GSSx8, GSSx12, and GSSx16 can be obtained, and DNA fragments having various numbers of repeats such as GSSx11, GSSx14, and GSSx15 can be obtained by mixing these with GSSx7 and conducting ligation reaction.

A linker sequence having a final target number of repeats was obtained by inserting a DNA fragment having each target number of repeats (the final target number of repeats-the number of repeats inserted into the adaptor vector) into the BamHI site or the MorI site of the GSS adaptor vector, the SAGG adaptor vector, or the GGGGS adaptor vector. These linker sequences were isolated by double digestion with XmaI and BamHI or XmaI and MorI.

To insert a final repeat fragment in place of a 95aa linker of the ND1-95-ND1 sequence, plasmids TALE24-scND1(bam)/pcDNA3.1s and TALE24-scND1(mro)/pcDNA3.1s in which a restriction enzyme site of BamHI or MorI was inserted immediately before the C-terminal-side ND1 in a TALE24-ND1-95-ND1 expression plasmid were prepared. Primers used in the preparation of these plasmids are shown in Table 5.

**[Table 5]**

| **Products** | **Template** | **Forward Primer** | **SEQ ID NO** | **Reverse Primer** | **SEQ ID NO** |
|---|---|---|---|---|---|
| TALE24-scND1 (bam)/pcDNA3. 1s | TALE24-scND1/pcDNA3.1s | bamND1_F | 56 | 95aa_R _inf_ bamND1 | 57 |
| TALE24-scND1 (mro) /pcDNA3. 1s | TALE24-scND1/pcDNA3.1s | mroND1_F | 58 | 95aa_R_inf_ mroND1 | 59 |

Next, after double digestion was conducted on each plasmid with XmaI and BamHI or XmaI and MorI, the 5' end was dephosphorylated with alkaline phosphatase. Thereafter, ligation reaction was conducted together with the linker sequences having the final target numbers of repeats prepared beforehand to eventually obtain TALE24-ND1-GSSx32-ND1, TALE24-ND1-SAGGx24-ND1, and TALE24-ND1-GGGGSx19-ND1. The base sequence and amino acid sequence of each fusion nuclease domain are shown in SEQ ID NOs: 88 to 93. The TALE repeat for each target was inserted into each plasmid, which was then subjected to the SSA assay.

### (6) Quantification of double-strand break activities on DNA by the SSA assay

1x10⁴ HEK293 T-cell grown in a DMEM medium containing 10% FBS were seeded in each well of a 96 well plate a day before transfection. Then, 33 ng of a TALE-L expression plasmid, 33 ng of a TALE-R expression plasmid, and 33 ng of an EGxxFP reporter plasmid were introduced into HEK293 T-cells by using Lipofectamine 3000 (Thermo Fisher Scientific). In the case of reducing the type or the introduction amount of the introduced plasmid, the amount was supplemented with pBluescript II (SK+) (Stratagene, La Jolla, CA, USA) such that the entire amount of the plasmid introduced became 100 ng.

The cells were cultured for 48 hours after the transfection, and the amount of fluorescence by the EGFP protein per unit area of each well was measured by using a plate reader. To reduce variations due to localization of the cells in each well, the fluorescence measurement was conducted at 16 points in each well, and an average value of the measurements was taken as the amount of fluorescence. The average values and the standard deviations of values of three independent wells were graphed.

### 2. Results

### (1) Check on activity of scFokI

First, the scFokI activities of reported molecules were checked as monomer-type nucleases that lacked the base recognizing activity. As TALE target sequences, the Right TALE sequence (tACAGAAGCGGGCAAAGG/corresponding to positions 119 to 136 of SEQ ID NO: 94) and the Left TALE sequence (tATGTACGCCTCCCTGGG/corresponding to positions 85 to 102 of SEQ ID NO: 94) of TALEN for human adenomatous polyposis coli (APC) genes, the intracellular activities of which had already been confirmed in NPL (the above-described document of Sakuma et al.) were used. As the positive control, Platinum TALEN (APC-R)+Platinum TALEN (APC-L) was placed, and evaluation was conducted by the SSA assay method in the HEK293 T-cells. As a result, there was no significant difference in the activities of Platinum TALE (+136/+63 scaffold, APC-R)-scFokI and Platinum TALE (+136/+63 scaffold, APC-L)-scFokI as compared with the case of only the reporter of the SSA assay (Fig. 1). In view of this, with the expectation of improvement of the activities, Platinum TALE (+136/+63 scaffold, APC-R)-scFokI-12 and Platinum TALE (+136/+63 scaffold, APC-L)-scFokI-12 in which a linker sequence between FokIs was changed to a flexible linker of GGGGSx12 were prepared and evaluated in the same manner. However, no activity was observed like scFokI (Fig. 1). It was determined that scFokI had very low cleavage activity and was difficult to utilize for efficient genome editing in human cells.

### (2) Preparation of scND1 and checking of activity

Next, the same study was conducted on ND1/ND2 which were found as substitutional factors for FokI, following the structure of scFokI.

Expression plasmids of TALE(APC-L)ND1 mono, TALE(APC-R)ND1 mono, TALE(APC-L)ND1-60-ND1, TALE(APC-L)ND1-95-ND1, TALE(APC-L)ND1-120-ND1, TALE(APC-L)ND1-180-ND1, TALE(APC-L)ND2 mono, TALE(APC-R)ND2 mono, TALE(APC-L)ND2-60-ND2, TALE(APC-L)ND2-95-ND2, TALE(APC-L)ND2-120-ND2, and TALE(APC-L)ND2-180-ND2 were prepared by using +136/+63 Platinum TALE as a scaffold (Fig. 2), and were evaluated by the SSA assay in the 293 T-cells. As a result, normal TALE-ND1(TALE(APC-L)ND1 mono+TALE(APC-R)ND1 mono) had significantly low activity as compared with normal TALE-ND2(TALE(APC-L)ND2 mono+TALE(APC-R)ND2 mono); however, when these were linked to form a single-chain, surprisingly both ND1s exhibited excellent activity, but both ND2s did not exhibit activity (Fig. 3). Moreover, in the case where TALE was changed to +153/+47 scaffold, TALE-scND1 exhibited attenuated activity as a whole, but TALE-ND1-95-ND1 held higher activity as compare with +136/+63 scaffold (Fig. 4). Although, the above findings regarding scND1 were results obtained in TALE repeats for APC-L, the TALE-ND1-95-ND1 also similarly exhibited activities in other 5 types of target sequences, specifically, the Right TALE sequence (tCGACATAGTGATTAGGA/corresponding to positions 357 to 374 of SEQ ID NO: 96) and the Left TALE sequence (tGAACCAGGCTATGACC/corresponding to positions 324 to 340 of SEQ ID NO: 96) of TALEN designed on APC-R, human hypoxanthine phosphoribosyl transferase 1 (HPRT1) loci (the above-described document of Sakuma et al.), as well as the Right TALE sequence (tGCCCAGAAGACTCCCG/corresponding to positions 163 to 179 of SEQ ID NO: 95) and the Left TALE sequence (tGATCTGCAAGTCGAGGC/corresponding to positions 130 to 147 of SEQ ID NO: 95) of TALEN designed on human Rosa26 locus (Sato et al. (2015) Stem Cell Reports, 14;5(1), 75-82) (Fig. 5). From the above results, it was revealed that as the linker sequence between ND1s, the sequence of 95 amino acids found in HTS was preferable. In the following experiments, TALE of +153/+47 scaffold which exerts particularly high activity in combination with ND1-95-ND1 was mainly used.

### (3) Optimization of the structure of TALE-scND1

Next, the influence of the C-terminal sequence length of TALE scaffold +153/+47 on activities were studied. First, when the C-terminal sequence was extended (63, 75, 90, 105, 120, 135, 150, 165, and 180 residues) in TALE(Rosa26-L)scND1 and TALE(Rosa26-R)scND1, no change in activity depending on the number of residues was observed (Fig. 6). In contrast, when the number of residues was reduced to 36, 24, 12, 0, while the activity was attenuated with 0, 12, and 36 residues depending on the target sequence, the activity with 24 residues was stably equal to or greater than that with the original 47 residues (Fig. 7).

In "(2) Preparation of scND1 and checking of activity", the linker length between ND1s was changed to 60, 95, 120, and 180 residues, and a result that 95 residues was optimum was obtained. However, the linker of 95 residues was a unique sequence obtained from HTS, and the other linkers of 60, 120, and 180 residues were composed of repeating flexible linkers of GGGGS sequence. Hence, it was unclear whether the chain length of 95 residues was optimum as a linker, or whether the HTS sequence was optimum. In view of this, three types of flexible linkers (GSSx32, SAGGx24, and GGGGSx19) were prepared, and were inserted as linkers of scND1 used in "(2) Preparation of scND1 and checking of activity" (Fig. 8), and the activities were compared with the case where the HTS95 sequence was used as a linker (Fig. 9). As a result, when the HTS95 sequence was used, favorable activities as a whole were observed. In the case where the APC gene was a target, activities similar to those with the HTS95 sequence were observed in the other linkers as well.

### [Industrial Applicability]

The artificial nucleic-acid cleaving enzyme using the fusion nuclease domain of the present invention has a site-specific DNA double-strand break activity with one molecule, and makes it possible to easily edit DNA as compared with the conventional TALENs using a pair of two molecules binding to the respective chains of DNA. The artificial nucleic-acid cleaving enzyme comprising the fusion nuclease domain of the present invention can be utilized as an excellent genome editing tool in a wide variety of industrial fields such as medicine, agriculture, and industry.

## Claims

1. A fusion nuclease domain comprising two nuclease domains 1 bound via a linker.

2. A site-specific DNA-cleaving enzyme comprising a DNA-binding domain and the fusion nuclease domain according to claim 1.

3. A nucleic acid encoding the fusion nuclease domain according to claim 1 or the site-specific DNA-cleaving enzyme according to claim 2.

4. A vector comprising the nucleic acid according to claim 3 or a translation product thereof.

5. A method for producing a cell in which a target DNA is modified, comprising introducing a molecule selected from the following (a) to (c) into a cell:
(a) the site-specific DNA-cleaving enzyme according to claim 2,
(b) a nucleic acid encoding the site-specific DNA-cleaving enzyme of (a), and
(c) a vector containing the nucleic acid according to (b) or a translation product thereof.

6. A kit for modifying a target DNA, comprising a molecule selected from the following (a) to (c):
(a) the site-specific DNA-cleaving enzyme according to claim 2,
(b) a nucleic acid encoding the site-specific DNA-cleaving enzyme of (a), and
(c) a vector containing the nucleic acid according to (b) or a translation product thereof.
